# Europäisches Patentamt

# European Patent Office

# Office européen des brevets

(11) Veröffentlichungsnummer: **0 100 522**
**B1**

## EUROPÄISCHE PATENTSCHRIFT

(45) Veröffentlichungstag der Patentschrift:
**02.11.88**

(21) Anmeldenummer: **83107375.4**

(22) Anmeldetag: **27.07.83**

(51) Int. Cl.⁴: **C 07 K 15/14, C 07 K 15/06,
G 01 N 33/68, A 61 K 37/02**

(54) **Neues Protein (PP17), Verfahren zu seiner Anreicherung und Gewinnung sowie seine Verwendung.**

(30) Priorität: **30.07.82 DE 3228503**

(43) Veröffentlichungstag der Anmeldung:
**15.02.84 Patentblatt 84/7**

(45) Bekanntmachung des Hinweises auf die Patenterteilung:
**02.11.88 Patentblatt 88/44**

(84) Benannte Vertragsstaaten:
**AT BE CH DE FR GB IT LI LU NL SE**

(56) Entgegenhaltungen:
**EP-A-0 009 715**
**EP-A-0 033 000**
**DE-A-2 640 387**
**US-A-2 042 430**

**CHEMICAL ABSTRACTS, Band 86, Nr. 19, 9. Mai
1977, Seite 189, Zusammenfassung Nr. 135224x,
Columbus, Ohio, US; H. BOHN et al.: "Isolation and
characterization of a new tissue protein (PP7) from
human placenta" & ARCH. GYNAEKOL. 1977, 222(1),
5-13**

(73) Patentinhaber: **BEHRINGWERKE Aktiengesellschaft,
Postfach 1140, D-3550 Marburg 1 (DE)**

(72) Erfinder: **Bohn, Hans, Dr., Oberer Eichweg 26,
D-3550 Marburg/Lahn (DE)**
Erfinder: **Winckler, Wilhelm, Untere Bergstrasse
21, D-3556 Wenkback (DE)**

(74) Vertreter: **Meyer- Dulheuer, Karl- Hermann, Dr.,
HOECHST Aktiengesellschaft Zentrale
Patentabteilung Postfach 80 03 20, D-6230
Frankfurt/Main 80 (DE)**

## Beschreibung

Die Erfindung betrifft ein neues Protein (PP$_{17}$), ein Verfahren zu seiner Anreicherung und Gewinnung aus dem Extrakt menschlicher Plazenten sowie seine Verwendung.

Im Extrakt aus menschlichen Plazenten wurde bereits eine Reihe löslicher Proteine, die aus diesem Gewebe stammen, nachgewiesen (Bohn H., Placental and Pregnancy Proteins, in: Carcino-Embryonic Proteins, Vol. 1, Ed. F.G. Lehmann, Elsevier/North-Holland Biomedical Press, 1979),

Die vorliegende Erfindung beschreibt die Isolierung und Charakterisierung eines neuen löslichen Plazenta-Proteins, genannt PP$_{17}$.

Aus einer ausgewachsenen menschlichen Plazenta (600 g) lassen sich mit physiologischer Salzlösung im Durchschnitt 2,5 mg dieses Proteins extrahieren.

Extrakte anderer Organe des Menschen (zum Beispiel aus Herz, Lunge, Haut, Magen, Niere, Uterus, Leber, Milz, Nebenniere, Colon und Blase) enthalten dieses Protein nicht oder in wesentlich geringerer Konzentration. Auch im Serum von anderen Körperflüssigkeiten des Menschen kommt PP$_{17}$ normalerweise nicht oder nur in Spuren (< 1 mg/l) vor.

Gegenstand der Erfindung ist das Protein PP$_{17}$, gekennzeichnet durch

a) eine elektrophoretische Beweglichkeit im Bereich zwischen $\beta_1$- und $\alpha_2$-Globulinen,

b) einen isoelektrischen Punkt von 5,25 $\pm$ 0,20,

c) einen Sedimentationskoeffizienten S$_{20,w}$ von 2,7 $\pm$ 0,1 S,

d) ein im Natriumdodecylsulfat (SDS)-haltigen Polyacrylamidgel bestimmtes Molekulargewicht von 38 000 $\pm$ 2 000,

e) einen Extinktionskoeffizienten E$^{1\%}_{1cm}$ (280 nm) von 8,5 $\pm$ 0,4,

f) einen Kohlenhydratanteil von 2,1 $\pm$ 0,9 % (Mannose 0,3 $\pm$ 0,2 %, Galactose 0,4 $\pm$ 0,2 %, Xylose 0,2 $\pm$ 0,1 %, N-Acetyl-Glucosamin 1,0 $\pm$ 0,3 %, N-Acetyl-Neuraminsäure 0,2 $\pm$ 0,1 %).

Die Aminosäurenzusammensetzung von PP$_{17}$ ist in der nachstehenden Tabelle angegeben:

Aminosäurenzusammensetzung von PP$_{17}$

| | Reste pro 100 Reste (Mol %) | Variations-Koeffizient (%) |
|---|---|---|
| Lysin | 5,86 | 5,52 |
| Histidin | 2,16 | 36,37 |
| Arginin | 5,30 | 2,90 |
| Asparaginsäure | 7,44 | 2,59 |
| Threonin | 4,41 | 9,80 |
| Serin | 9,14 | 4,73 |
| Glutaminsäure | 18,52 | 2,94 |
| Prolin | 4,41 | 5,13 |
| Glycin | 4,93 | 2,92 |
| Alanin | 8,56 | 1,55 |
| Cystin /2 | 0,75 | 3,58 |
| Valin | 6,91 | 3,53 |
| Methionin | 1,46 | 7,89 |
| Isoleucin | 2,53 | 4,70 |
| Leucin | 11,39 | 1,55 |
| Tyrosin | 1,43 | 11,97 |
| Phenylalanin | 2,79 | 7,29 |
| Tryptophan | 1,97 | 8,41 |

Zur Erläuterung der kennzeichnenden Merkmale des Gewebsproteins sei folgendes ausgeführt:

Die Untersuchung der elektrophoretischen Beweglichkeit erfolgte in der Mikromodifikation mit dem Gerät Microzone R 200 von Beckman Instruments auf Zelluloseazetatfolien (Fa. Sartorius) unter Verwendung von Natriumdiäthylbarbiturat-Puffer, pH 8,6.

Die Bestimmung des isoelektrischen Punktes wurde mit einer Säule (440 ml) der Firma LKB, Stockholm, durchgeführt. Das sogenannte Ampholin (R)-Gemisch hatte bei der Untersuchung des Glykoproteins einen pH-Bereich von 4,0 bis 6,0.

Per Sedimentationskoeffizient wurde in einer analytischen Ultrazentrifuge der Firma Beckman (Spinco-Apparatur, Modell E) bei 60 000 UpM in Doppelselektorzellen mit Hilfe der UV-Scanner Technik bei 280 nm bestimmt. Als Lösungsmiitel diente ein 0,05 M Phosphatpuffer (pH 6,8), der 0,2 Mol/l NaCl enthielt. Die Proteinkonzentration wurde auf eine optische Dichte von etwa 3 eingestellt. Der Sedimentationskoeffizient wurde auf die Basis von Wasser bei 20°C umgerechnet.

Zur Bestimmung des Molekulargewichtes im SDS-PAA-Gel wurde ein Gel mit 7,5 % Polyacrylamid (PPA), das 0,1 % Natriumdodecylsulfat (SDS) enthielt, verwendet. Als Vergleichssubstanz dienten humanes Plazentalactogen (HPL) und Humanalbumin sowie dessen Aggregate.

Zur Bestimmung des Extinktionskoeffizienten wurde die Substanz 0,10 %-ig (g/100 ml) in destilliertem Wasser gelöst das durch Zusatz von Ammoniumbicarbonat auf pH 7,0 eingestellt worden war.

Die Analyse der Kohlenhydrate wurde wie folgt durchgeführt: Nach Hydrolyse der glykosidischen Bindungen wurden die freigesetzten Neutralzucker als Boratkomplexe über eine Anionenaustauschersäule getrennt (Y.C. Lee et al., Anal. Biochem. 27, (1969) 567), Im Eluat durch Zumischung von Cu(I)bicinchoninatreagenz (K. Mopper und M. Gindler, Anal. Blochem., 56, (1973), 440) angefärbt und unter Verwendung von Rhamnose als internem Standard quantitativ bestimmt. Die Aminozucker wurden durch ihre Reaktion mit Ninhydrin nachgewiesen und bestimmt. Der Neuraminsäuregehalt ist mit der Methode nach Warren (Methods in Enzymology, Vol. VI, 1963) 463 - 465) ermittelt worden.

Die Aminosäurenanalyse wurde nach S. Moore, D.H. Spackman, W.H. Stein, Anal. Chem., 30, (1958), 1185, unter Verwendung des Flüssgigkeitschromatographen Multichrom B der Firma Beckman durchgeführt. 1/2 Cystin wurde nach Oxydation der Proteine mit Perameisensäure (S. Moore et al., Anal. Chem., 30, (1958) 1185,) und nachfolgender Chromatographie (S. Moore, J. Biol. Chem., 238, (1963) 235) als Cysteinsäure bestimmt. Der Tryptophangehalt ist mit der direkten photometrischen Bestimmung nach H. Edelhoch, Biochemi, 6, (1967) 1948 ermittelt worden.

$PP_{17}$ hat folgende Eigenschaften, die sich in einem Verfahren zu seiner Isolierung verwenden lassen, indem diesen Eigenschaften entsprechende Maßnahmen getroffen werden:

1. Mit Ammoniumsulfat wird es bei pH 5 - 8 und 30 - 50 % Sättigung aus wässrigen Lösungen gefällt.

2. Mit wasserlöslichen Acridinbasen, zum Beispiel 2-Äthoxi-6,9-diaminoacridinlactat wird es bei pH-Werten zwischen 7 - 9 und einer Konzentration der Base von 0,4 bis 0,8 g/100 ml präzipitiert, nicht oder kaum dagegen bei pH 6,0, wenn die Konzentration der Base $\leq$ 0,4 g/100 ml beträgt.

3. Bei Fällungen mit Äthanol bleibt es in verdünnten Salzlösungen (zum Beispiel in 1 - 2 % NaCl-Lösung) bei pH 7,0 bis zu einer Konzentration von 20 Vol.-% Alkohol zur Hauptsache im Überstand.

4. Bei der elektrophoretischen Auftrennung zeigt $PP_{17}$ bei pH 8,0 eine Beweglichkeit, die zwischen der von $\beta_1$- und $\alpha_2$-Globulinen liegt.

5. Bei einer isoelektrischen Fokussierung erscheint es im pH-Bereich von 5,1 bis 5,4 mit Maximum zwischen 5,2 und 5,3.

6. Bei der Dialyse gegen einen 0,5 M Glycin-HCl-Puffer (pH 2,5) ist das Protein $PP_{17}$ stabil und wird immunchemisch nicht verändert im Gegensatz zu einigen anderen Plazentaproteinen (wie zum Beispiel $PP_9$), die unter diesen Bedingungen ihre immunchemische Reaktivität verlieren.

7. Bei der Gelfiltration (Sephadex® erscheint $PP_{17}$ im Bereich der Proteine mit Molekulargewichten von 20 000 bis 60 000.

8. $PP_{17}$ läßt sich an schwach basische Ionenaustauscher wie zum Beispiel DEAE-Cellulose oder DEAE-Sephadex bei niedriger Leitfähigkeit (etwa 0 - 2 mS) und neutral oder schwach alkalischem pH-Wert (etwa pH 7 bis 9) adsorbieren und wird erst unter Verwendung stärker konzentrierter Salzlösungen (0 - 2 % (g/100 ml) NaCl-Lösungen) vom Ionenaustauscher wieder eluiert.

9. $PP_{17}$ kann aus seiner wässrigen Lösung durch Immunadsorption angereichert und isoliert werden.

Gegenstand der Erfindung ist ein Verfahren zur Gewinnung des $PP_{17}$, dadurch gekennzeichnet, daß der Extrakt aus menschlichen Plazenten unter Verwendung der obenstehenden Eigenschaften fraktioniert wird.

Neben Ammoniumsulfat können selbstverständlich auch andere in der präparativen Biochemie üblicherweise eingesetzte Neutralsalze zur Ausfällung des $PP_{17}$ verwendet werden. Neben einer Acridinbase ist auch ein wasserlösliches Derivat einer Chinolinbase, wie sie für Proteinfraktionierungen bekannt sind, im Rahmen des erfindungsgemäßen Verfahrens einsetzbar. Entsprechend seinem elektrophoretischen Verhalten wie seinem Molekulargewicht sind zur Isolierung des Proteins auch andere Maßnahmen brauchbar, die geeignet sind, ein Protein mit den angegebenen Eigenschaften von anderen Proteinen abzutrennen. Hierzu können die verschiedenen Methoden der Gelfiltration, Gelchromatographie oder Ultrafiltration oder auch die Eigenschaft des $PP_{17}$, aus verdünnten Pufferlösungen an schwach basische Ionenaustauscher gebunden und hiervon mit stärker konzentrierten Salzlösungen wieder eluiert werden zu können, verwendet werden.

Durch eine zweckmäßige Kombination der genannten Maßnahmen, die eine Anreicherung des $PP_{17}$ oder eine Abtrennung dieses Proteins von anderen Proteinen bewirken, kann das $PP_{17}$ isoliert werden.

Demzufolge ist der Gegenstand der vorliegenden Erfindung in den einzelnen Schritten zur Anreicherung des $PP_{17}$ und in dem durch Kombination der Maßnahmen zur Anreicherung sich ergebenden Verfahren zur Reinigung des $PP_{17}$ zu sehen.

Das Beispiel beschreibt die Isolierung von $PP_{17}$ unter Verwendung der Methode der Immunadsorption. Man könnte zur Immunadsorption direkt den wässrigen Extrakt aus menschlichen Plazenten einsetzen. Da die Konzentration von $PP_{17}$ im Extrakt relativ gering ist, ist es zweckmäßig, durch eine Vorfraktionierung des Extraktes das Protein $PP_{17}$ erst spezifisch anzureichern.

Dazu können im Grunde genommen die verschiedensten Methoden, die sich zur Fraktionierung von Proteinen in größerem Maßstab eignen, herangezogen werden, so zum Beispiel die fraktionierte Fällung mit neutralen Salzen, organischen Kationen oder Alkohol sowie die Auftrennung durch Gelfiltration oder Ionenaustauscherchromatographie.

Zweckmäßig hat sich in vielen Fällen bei der Isolierung von Plazentaproteinen eine kombinierte Fraktionierung mit wasserlöslichen Acridinbasen und Ammoniumsulfat erwiesen. Sie wurde auch bei der Isolierung von $PP_{17}$ angewandt und ist im Beispiel unter A)

beschrieben.

Zur weiteren Anreicherung wurde anschließend ein Teil der Begleitproteine durch Fällung mit Alkohol abgeschieden.

Die im Beispiel angegebenen Schritte zur Anreicherung sind aber nicht zwingend und müssen auch keinesfalls in der dort beschriebenen Reihenfolge durchgeführt werden.

Auch der Schritt der Immunadsorption könnte durch Anwendung anderer Trennmethoden, zum Beispiel durch präparative Elektrophorese und isoelektrischer Fokussierung nach Anwendung einer Dialyse gegen einen sauren Puffer von pH 2 - 4, vorzugsweise 0,5 M Glycin-HCl (pH 2,5), ersetzt werden.

Zur Hochreinigung von PP$_{17}$ haben sich Gelfiltration an Sephadex G-100, Ionenaustauschromatographie an DEAE-Sephadex und inverse Immunadsorption als brauchbar erwiesen.

Zum Nachweis des PP$_{17}$ etwa in einer Fraktion aus einer Trennoperation können neben den angegebenen Parametern auch immunchemische Methoden dienen, da PP$_{17}$ antigene Eigenschaften hat. Bei der Immunisierung von Tieren mit diesem Protein werden spezifische Antikörper gebildet.

Ein fur diesen Zweck brauchbares Antiserum kann folgendermaßen gewonnen werden:

Bei der Fraktionierung des Plazentenextraktes mit 2-Äthoxy-6,9-Diaminoacridinlactat und Ammoniumsulfat nach H. Bohn, (Arch. Gynäkol., 210, (1971), 440) geht PP$_{17}$ zur Hauptsache in die Plazentafraktion III. Diese Fraktion wird 14 Stunden gegen einen 0,5 M Glycin-HCl-Puffer (pH 2,5) dialysiert, dann neutralisiert und gegen physiologische Kochsalzlösung dialysiert. Durch Immunisieren von Kaninchen mit dieser Fraktion wird ein polyvalentes Antiserum erhalten, mit dem PP$_{17}$ nachgewiesen werden kann. Dieses Antiserum kann durch Absorption mit normalem menschlichen Serum und solchen Plazentafraktionen, die PP$_{17}$ nicht enthalten, gegen das Antigen PP$_{17}$ weitgehend spezifisch gemacht werden. Dieses spezifische Antiserum kann einerseits zum immunologischen Nachweis des PP$_{17}$, andererseits zur Herstellung eines Immunadsorbens dienen, das zur Anreicherung und Isolierung von PP$_{17}$ eingesetzt werden kann.

Mit Hilfe des nach der vorliegenden Anmeldung gewonnenen hochgereinigten PP$_{17}$ lassen sich durch Immunisierung von Tieren nach bekannten Methoden dann direkt monospezifische Antiseren herstellen.

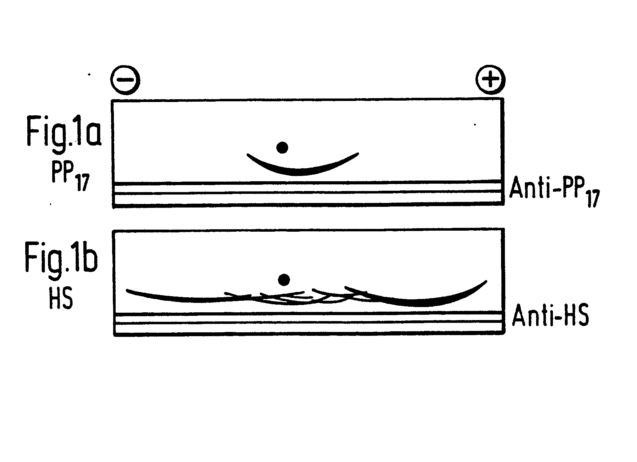

Die immunologische Reaktion von PP$_{17}$ mit einem spezifischen Antiserum vom Kaninchen nach Auftrennung im elektrischen Feld in Agarhaltigem Gel zeigt Abbildung 1a. Abbildung 1 b bringt zum Vergleich dazu die Auftrennung der Proteine des Serums, sichtbar gemacht durch deren Immunreaktion mit einem Antiserum vom Kaninchen gegen Humanserum (HS).

Zum immunologischen Nachweis von PP$_{17}$ kann auch die Geldiffusionstechnik nach Ouchterlony (vgl. Schultze and Heremans, Molecular Biology of Human Proteins, Vol. 1, p. 134) oder noch empfindlichere Methoden wie Radioimmunoassays und Enzymimmunoassays herangezogen werden.

PP$_{17}$ ist ein mehr oder weniger plazenta-"spezifisches" Protein. Der Nachweis und die Bestimmung solcher Proteine hat diagnostische Bedeutung einerseits zur Überwachung von Schwangerschaften, andererseits zum Nachweis, speziell von trophoblastischen, aber auch von nicht-trophoblastischen Tumoren sowie zur Überwachung des Krankheitsverlaufs und zur Kontrolle der Therapie bei solchen Erkrankungen.

PP$_{17}$ kann also verwendet werden, um Antiseren herzustellen, die dazu dienen können, PP$_{17}$ nachzuweisen und zu bestimmen.

Die Erfindung wird am nachstehenden Beispiel erläutert:

**Beispiel**

A) Extraktion der Plazenten und Fraktionierung des Extraktes mit Rivanol und Ammoniumsulfat

1000 kg tiefgefrorene menschliche Plazenten wurden im Schneidmischer zerkleinert und mit 1000 l einer 0,4 % (g/100 ml) Kochsalzlösung extrahiert. Der Extrakt ist dann, nach Abtrennung des Geweberückstandes durch Zentrifugation, mit 20 %-iger Essigsäure auf pH 6,0 eingestellt und unter Rühren mit 200 l einer 3 %-igen (g/100 ml) Lösung von 2-Äthoxy-6,9-Diamino-acridin-Lactat (Rivanol®, Hoechst AG) versetzt werden. Der Niederschlag wurde abzentrifugiert und verworfen. Der Überstand wurde mit 1 % (g/100 ml) Betonit A (Firma Erbslöh & Co., Geisenheim/Rhein) versetzt und durch Zugabe von 2 N NaOH auf pH 7,0 eingestellt und filtriert. Das Filtrat ist unter Rühren langsam mit 30 % (g/100 ml) Ammoniumsulfat versetzt worden; das Plazentaprotein PP$_{17}$ fiel dabei, zusammen mit anderen Proteinen, aus. Der Niederschlag wurde abfiltriert; erhalten wurden etwa 12 kg einer feuchten Paste, die im nachfolgenden als Fraktion A bezeichnet wird.

B) Fraktionierung mit Äthanol

500 g der Fraktion A wurden in 400 ml Wasser gelöst und bei 4° C gegen physiologische Kochsalzlösung dialysiert. Nach der Dialyse wurde die Leitfähigkeit der Lösung durch Zugabe von 5 % (g/100 ml) NaCl-Lösung auf 15 mS eingestellt. Die Lösung ist dann auf 0° C abgekühlt und unter Rühren langsam mit 96 %-igem Äthanol bis zu einer Endkonzentration des Alkohols von 20 % (g/100 ml) versetzt worden. Der Niederschlag wurde durch Zentrifugation entfernt. Der Überstand ist zunächst gegen Wasser, dann gegen einen 0,1 M Tris-HCl-Puffer (pH 8,0) der 1,0 Mol/l NaCl und 0,1 % (g/100 ml ) NaN$_3$ enthielt (Pufferlösung II), dialysiert worden. Anschließend wurden die Proteine durch Zugabe von 38 % (g/100 ml) festem Ammoniumsulfat ausgefällt. Der Niederschlag wurde in Wasser gelöst und gegen Pufferlösung II dialysiert. Erhalten wurden ca. 850 ml einer Lösung (Fraktion B), die im Durchschnitt 35 mg PP$_{17}$ enthielt.

C) Anreicherung von PP$_{17}$ durch Immunadsorption

1. Herstellung des Immunadsorbens

400 ml eines Anti-PP$_{17}$-Serums vom Kaninchen wurden gegen 0,02 M Phosphatpuffer (pH 7,0) dialysiert und zur Abtrennung der Immunglobuline an DEAE-Cellulose chromatographiert. Die Immunglobulinfraktion (5,95 g Protein) wurde dann mit 595 g besonders gereinigter Agarose in Kugelform (Sepharose® 4 B der Pharmacia, Uppsala, Schweden), die mit 74,4 g Bromcyan aktiviert worden war, umgesetzt und so kovalent an einen Träger gebunden.

Das Verfahren ist beschrieben von R. Axen, J. Porath, S., Ernbach, Nature, 214, (1967) 1302.

Mit Hilfe eines auf diese Weise hergestellten Immunadsorbens konnte das Plazentaprotein PP$_{17}$ aus dessen Lösung, insbesondere aus PP$_{17}$-angereicherten Plazentenfraktionen isoliert werden.

2. Durchführung der Immunadsorption

Das Immunadsorbens wurde in Pufferlösung II (0,1 M Tris-HCl-Puffer, pH 8,0 mit 1,0 Mol/l NaCl und 0,1 % NaN$_3$) suspendiert, dann in eine Chromatographiesäule gefüllt (5,0 x 21 cm) und mit Pufferlösung 11 nachgespült. Dann wurde die halbe Menge der Fraktion B auf die Säule aufgetragen, wobei PP$_{17}$ immunadsorptiv gebunden wurde. Die Säule wurde dann gründlich mit Puffer II gespült. Anschließend ist das adsorbierte Protein mit etwa 600 ml 3 M Kaliumrhodanid-Lösung von der Säule eluiert worden. Die PP$_{17}$-haltigen Eluate wurden gegen Pufferlösung II dialysiert und im Ultrafilter auf ca. 15 ml eingeengt. Ausbeute pro Adsorption ca. 6 mg PP$_{17}$.

Das Adsorbens in der Säule wurde unmittelbar nach der Elution von PP$_{17}$ wieder mit Pufferlösung I neutralisiert und gründlich gewaschen; es ist dann erneut zur immunadsorptiven Bindung von PP$_{17}$ eingesetzt worden.

D) Hochreinigung von PP$_{17}$

Das durch Immunadsorption gewonnene Protein war häufig noch durch unspezifisch gebundene Serumproteine und andere Plazentaproteine verunreinigt. Die Abtrennung der Hauptmenge der Serum-Begleitproteine gelang durch Gelfiltration an Sephadex G-100. Die restlichen Begleitproteine wurden dann durch inverse oder negative Immunadsorption, das heißt mit Hilfe von trägergebundenen Antikörpern gegen die noch als Verunreinigung vorliegenden Proteine, entfernt. Im wesentlichen handelte es sich dabei um Humanes Plazenta-Lactogen (HPL) und um Immunglobuline.

Ein zuweilen im PP$_{17}$-Präparat noch in geringer Menge vorhandenes Erythrozytenprotein konnte schließlich durch Chromatographie an DEAE-Sephadex entfernt werden. Das PP$_{17}$ wurde dazu in 0,01 M Tris-HCl-Puffer (pH 7,0) auf die Säule aufgebracht und adsorbiert und dann durch einen linearen Salzgradienten von 0 - 2 % (g/100 ml) NaCl eluiert. Das Erythrozytenprotein wurde dabei stärker am Ionenaustauscher adsorbiert und erst nach PP$_{17}$ von der Säule eluiert.

**Patentansprüche**

1. Protein PP$_{17}$, gekennzeichnet durch
a) eine elektrophoretische Beweglichkeit im Bereich zwischen $\beta_1$-und $\alpha_2$-Globulinen,
b) einen isoelektrischen Punkt von 5,25 $\pm$ 0,20,
c) einen Sedimentationskoeffizienten $S^0_{20,w}$ von 2,7 $\pm$ 0,1 S,
d) ein im Natriumdodecylsulfat (SDS)-haltigen Polyacrylamidgel bestimmtes Molekulargewicht von 38 000 $\pm$ 2 000,
e) einen Extinktionskoeffizienten $E^{1\%}_{1cm}$ (280 nm) von 8,5 $\pm$ 0,4 und
f) einen Kohlenhydratanteil von 2,1 $\pm$ 0,9 % enthaltend 0,3 $\pm$ 0,2 % Mannose, 0,4 $\pm$ 0,2 % Galactose, 0,2 $\pm$ 0,1 % Xylose, 1,0 $\pm$ 0,3 % N-Acetyl-Glucosamin und 0,2 $\pm$ 0,1 % N-Acetyl-Neuraminsäure.

2. Verfahren zur Gewinnung des Proteins nach Anspruch 1, dadurch gekennzeichnet, daß ein Extrakt aus menschlichen Plazenten, der ein Protein mit den im Anspruch 1 angegebenen Parametern enthält, einer geeigneten Kombination von mehreren zur Isolierung von Proteinen bekannten Verfahrensmaßnahmen unterworfen wird, wobei jeweils das Material gewonnen wird, welches das Protein mit den angegebenen Eigenschaften enthält, und das Protein gewonnen wird.

3. Verwendung des Proteins nach Anspruch 1 zur Gewinnung von spezifischen Antikörperen gegen dieses Protein.

4. Verwendung des Proteins in immunologischen Methoden zum Nachweis und zur Bestimmung dieses Proteins.

**Claims**

1. The protein PP$_{17}$, which has
a) an electrophoretic mobility in the range between $\beta_1$- and $\alpha_2$-globulins,
b) an isoelectric point of 5.25 $\pm$ 0.20,
c) a sedimentation coefficient $S^0_{20,w}$ of 2.7 $\pm$ 0.1 S,
d) a molecular weight, determined in polyacrylamide gel containing sodium dodecyl sulfate (SDS), of 38,000 $\pm$ 2,000,
e) an extinction coefficient $E^{1\%}_{1cm}$ (280 nm) of 8.5 $\pm$ 0.4 and
f) a carbohydrate content of 2.1 $\pm$ 0.9 % containing 0.3 $\pm$ 0.2 % of mannose, 0.4 $\pm$ 0.2 % of galactose, 0.2 $\pm$ 0.1 % of xylose, 1.0 $\pm$ 0.3 % of N-acetyl-glucosamine and 0.2 $\pm$ 0.1 % of N-acetyl-neuramin acid.

2. The process for isolating the protein as claimed in claim 1, which comprises subjecting an extract from human placentas containing a protein with the parameters given in claim 1 to a suitable combination of several process measures known for the isolation of proteins, in each case the material containing the protein with the given properties being obtained, and isolating the protein.

3. The use of the protein as claimed in claim 1 for obtaining specific antibodies against this protein.

4. The use of the protein in immunological methods for the detection and determination of this protein.

## Revendications

1. Protéine PP$_{17}$, caractérisée par

a) une mobilité électrophorétique dans le domaine compris entre les globulines $\beta_1$ et $\alpha_2$,

b) un point isoélectrique de 5,25 $\pm$ 0,20,

c) un coefficient de sédimentation $S^0_{20,w}$ de 2,7 $\pm$ 0,1 S,

d) une masse moléculaire déterminée dans un gel de polyacrylamide contenant du docécylsulfate de sodium (SDS) de 38 000 $\pm$ 2 000,

e) un coefficient d'extinction $E^{1\%}_{1cm}$ (280 nm) de 8,5 $\pm$ 0,4, et

f) une teneur en hydrates de carbone de 2,1 $\pm$ 0,9 %, contenant (0,3 $\pm$ 0,2 % de mannose, 0,4 $\pm$ 0,2 % de galactose, 0,2 $\pm$ 0,1 % de xylose, 1,0 $\pm$ 0,3 % de N-acétyl-glucosamine et 0,2 $\pm$ 0,1 % d'acide N-acétyl-neuraminique).

2. Procédé de préparation de la protéine selon la revendication 1, caractérisé en ce qu'on soumet un extrait de placentas humains qui contient une protéine présentant les paramètres indiqués dans la revendication 1 à une combinaison appropriée de plusieurs mesures opératoires connues pour l'isolement de protéines, en obtenant à chaque fois la matière qui contient la protéine avec les propriétés indiquées, et en obtenant la protéine.

3. Utilisation de la protéine suivant la revendication 1 pour la préparation d'anticorps spécifiques contre cette protéine.

4. Utilisation de la protéine dans des méthodes immunologiques de détection et le dosage de cette protéine.

Fig.1a
PP₁₇                                    Anti-PP₁₇

Fig.1b
HS                                      Anti-HS